# EUROPÄISCHE PATENTSCHRIFT

(11) **EP 3 232 180 B1**
(45) Veröffentlichungstag und Bekanntmachung des Hinweises auf die Patenterteilung: **31.01.2018**
(21) Anmeldenummer: 16165648.3
(22) Anmeldetag: 15.04.2016
(51) Int. Cl.: G01N 21/27, G01N 21/53, G01N 33/00, G01N 21/3504, G01N 1/22, F16K 7/00

(54) **VORRICHTUNG ZUR OPTISCHEN IN-SITU GASANALYSE**
DEVICE FOR OPTICALLY IN-SITU GAS ANALYSIS
DISPOSITIF D'ANALYSE OPTIQUE IN SITU D'UN GAZ

(43) Veröffentlichungstag der Anmeldung: 18.10.2017
(73) Patentinhaber: SICK AG, 79183 Waldkirch (DE)
(72) Erfinder: Kaufmann, Jürgen, 79211 Denzlingen (DE); Schiffler, Ingo, 79100 Freiburg (DE); Brüchig, Florian, 79232 March (DE)

(56) Entgegenhaltungen:
- DE-A1- 19 704 960
- DE-A1-102008 044 171
- DE-A1-102014 002 087
- Anonymous: "Dichtung (Technik) - Wikipedia", , 20. September 2015 (2015-09-20), XP055297124, Gefunden im Internet: URL:http://web.archive.org/web/20150920015 156/https://de.wikipedia.org/wiki/Dichtung _(Technik) [gefunden am 2016-08-23]

## Beschreibung

Die Erfindung betrifft eine Vorrichtung zur optischen in-situ Gasanalyse gemäß dem Oberbegriff des Anspruchs 1.

Aus der DE 10 2008 044 171 A1 ist ein Abgassensor für ein Kraftfahrzeug bekannt, der in den Abgasstrang einschraubbar ist und dort optisch bestimmte Bestandteile messen kann. Der Sensor ist in einer Ausführungsform doppelwandig ausgebildet, mit einem metallischen Schutzrohr außen mit Öffnungen für den Durchtritt des Abgases und mit einem innenliegenden Gehäuse, dass zum Durchlassen des zu messenden Abgases porös ausgebildet.

Aus einem Wikipediaeintrag vom 2015-09-20 mit URL http://web.archive.org/web/ 20150920015156/https://de.wikipedia.org/wiki/Dichtung_(Technik) sind aufblasbare Dichtungen bekannt.

Aus DE 197 04 960 A1 ist es bekannt eine Rohrleitung provisorisch mit einem Ballon zu verschliessen.

Aus DE 10 2014 002 087 A1 ist eine spektrometrische Gasanalysevorrichtung bekannt, bei der der Messraum im Inneren eines Doppelrohres gelegen ist und Innen- und Aussenrohr gegeneinander verschiebbar sind, um Öffnungen in Innen-und Aussenrohr entweder durch Übereinanderschieben zu öffnen oder durch Verscheiben zu verschließen.

Mit derartigen Gasanalysevorrichtungen werden bestimmte Gasanteile, z. B. Schwefelwasserstoff, Kohlenmonoxid, SO2, NH3, NO NO2, HCl, HF oder dergleichen, mittels optischer Transmission oder Lichtstreuung gemessen. Zumeist wird dabei die Konzentration dieser Gasanteile ermittelt.

Anwendungsgebiete sind zum Beispiel Emissionsmessungen von Industrieanlagen, bei denen die Abgase in einem Abgaskanal auf ihren Gehalt bestimmter molekularer Verbindungen überwacht werden müssen. Häufig sind die Gasströme, denen die optoelektronische Vorrichtung ausgesetzt ist, um die gewünschten Gasanteile zu messen, durch hohe Partikelbelastungen, wie zum Beispiel Rauch, Stäube oder andere Aerosole, gekennzeichnet. Diese hohen Partikelbelastungen verursachen eine große Lichtabsorption und/oder eine hohe Lichtstreuung, die die eigentliche Messung stark behindert bis unmöglich macht. So hat beispielsweise Schwefelwasserstoff eine sehr breite Absorption wie auch ultrafeiner Staub. Es kann dann nicht mehr unterschieden werden, ob die Absorption vom Schwefelwasserstoff herrührt oder von dem Staub.

Zum Fernhalten derartiger Partikel, die die Messung stören, ist es bekannt (z.B. US 4,549,080) Filter vorzusehen, die aus einem Rohrstück aus porösem Material bestehen, in dessen Innerem sich die Messstrecke befindet. Aufgrund der porösen Struktur kann zwar das zu messende Gas in die Messstrecke gelangen, aber je nach Porengröße können Partikel, wie Rauch, Stäube oder Aerosole, abgehalten werden.

Nachteilig daran ist, dass solche in-situ Geräte von Zeit zu Zeit getestet, geprüft bzw. kalibriert werden müssen und zu diesem Zweck ein Prüfgas in die Messstrecke eingebracht werden muss. Dazu wird das Prüfgas in die Messstrecke eingeblasen. Die Messstrecke ist aber nicht hermetisch dicht, sondern das Prüfgas entweicht durch die Poren des Filters in den Abgaskanal. Für die Dauer der Kalibriermessungen muss daher eine ausreichende Menge an Prüfgas in die Messstrecke permanent mit ausreichendem Druck eingeblasen werden. Die für eine Kalibrierung benötigte Prüfgasmenge ist entsprechend hoch, was entsprechend hohe Kosten verursacht. Dieser Nachteil macht sich besonders bei langen Messstrecken mit entsprechend langem, porösem Filter bemerkbar.

Ausgehend von diesem Stand der Technik ist es Aufgabe der Erfindung, eine verbesserte Vorrichtung bereitzustellen, mit der der Verbrauch an Prüfgas reduziert werden kann.

Diese Aufgabe wird gelöst durch eine Vorrichtung mit den Merkmalen des Anspruchs 1.

Die erfindungsgemäße Vorrichtung zur optischen in-situ Gasanalyse umfasst
- ein Gehäuse,
- eine Messlanze, dessen eine erste Ende an das Gehäuse angeschlossen ist und die mit ihrem anderen zweiten Ende in das zu messende Gas hineinragt,
- einen im Gehäuse angeordneten Lichtsender, dessen Licht in die Messlanze geführt ist und von einem am zweiten Ende angeordneten Reflektor auf einen Lichtempfänger reflektiert wird und der Strahlengang eine optische Messstrecke innerhalb der Messlanze definiert,
- und eine Auswerteeinrichtung zur Auswertung von Lichtempfangssignalen des Lichtempfängers,
- und einen Prüfgasanschluss, über den die Messstrecke mit einem Prüfgas befüllbar ist,
- wobei die Messlanze ein Außenrohr aufweist und das Außenrohr Öffnungen für das zu messende Gas aufweist. Erfindungsgemäß sind die Öffnungen für die Prüfphase mit wenigstens einer Dichtung verschließbar, wobei die Dichtung durch Vergrößerung ihres Volumens die Öffnungen dichtend verschließt.

Mit dieser quasi "aufblasbaren" Dichtung werden die Öffnungen zur Messtrecke hin in einfacher Weise verschlossen, so dass kein Messgas mehr in die Messstrecke gelangen kann. Dann kann die Messstrecke mit Prüfgas geflutet werden. Eine definierte Undichtigkeit über ein Ablassventil oder eine definierte Undichtigkeit, die aber klein sein kann, ist dabei sinnvoll, um das nach Verschließen der Öffnungen noch vorhandene Messgas durch das Prüfgas aus der Messstrecke zu verdrängen. Das Prüfgas kann aber nur durch die kleine definierte Undichtigkeit entweichen und nicht mehr durch den Filter. Mit einem kleinen Überdruck in der entstehenden Messkammer in Zusammenhang mit konstantem Prüfgasfluss wird eine Prüfgasbefüllung der Messstrecke erreicht. Damit wird der Prüfgasverbrauch berechenbar und kann erheblich minimiert werden und ist auch weitestgehend unabhängig von der Länge der aktiven Messstrecke. Die Messstrecke wird darüber hinaus gleichmäßig mit Prüfgas befüllt. Der Prüfgasverbrauch ist konstant und vorhersagbar.

Solche Dichtungen sind einfach zu handhaben, bewirken keinen mechanischen Verschleiß und passen sich an unterschiedliche Geometrien in der Lanze selbständig an.

Diese Dichtungen eröffnen auch die Möglichkeit, durch periodische oder pulsierende Volumenveränderungen einen "Atmungseffekt" zu bewirken, so dass während der normalen Messphase ein besserer Gasaustausch zwischen Messstrecke und Abgaskanal erfolgt.

Die Dichtungen der erfindungsgemäßen Vorrichtung benötigen keine klassisch bewegten Teile, wodurch sich wenig Verschleiß und eine hohe Lebensdauer ergibt. Es sind nur wenige Bauteile notwendig. Die Dichtung ist konstruktiv sehr einfach und große Öffnungsflächen sind möglich, die einen besseren Gasaustausch ermöglichen. Durch die expansionsfähige Dichtung ist ein großer Toleranzausgleich möglich.

Den Prüfbetrieb könnte man automatisch in definierten Zeitintervallen durchführen oder aber durch manuelle Betätigung. Das würde sich in preislich abgestuften Varianten der erfindungsgemäßen Vorrichtung niederschlagen.

In einer Ausführungsform der Erfindung erfolgt Volumenänderung mit pneumatischen oder hydraulischen Mitteln, beispielsweise mittels einer zugeordneten Pumpe.

Um in bekannter Weise Partikel, wie Rauch, Stäube oder Aerosole, während der Messung aus der Messstrecke fern zu halten ist ein gasdurchlässiges Filter vorgesehen, durch das das Messgas in die Messstrecke gelangt. Das Filter besteht beispielsweise aus einem Rohrstück aus porösem Material und ist mit geeigneter Tragkonstruktion gehalten. Im Inneren des Filters befindet sich die Messstrecke. Aufgrund der porösen Struktur kann zwar das zu messende Gas in die Messstrecke gelangen, aber je nach Porengröße können Partikel abgehalten werden.

In Weiterbildung könnte das Prüfgas in einer Ausführungsform der Erfindung gleichzeitig als Treibgas für die Dichtungen benutzt werden.

Eine außen am Außenrohr anliegende Heizspirale kann den Eintritt von Wasser bei Anwendungen in einem nassen Messgas verhindern.

Im Folgenden wird die Erfindung anhand von Ausführungsbeispielen unter Bezugnahme auf die Zeichnung im Einzelnen erläutert. In der Zeichnung zeigen:
- Fig. 1: eine schematische Darstellung einer Ausführungsform der Vorrichtung zur optischen in-situ Gasanalyse in einem Gasstrom;
- Fig. 2: die Vorrichtung aus Fig. 1 in einem Schnitt entlang der Linie II-II;
- Fig. 3: die Vorrichtung aus Fig. 1 mit geschlossenen Öffnungen;
- Fig. 4: die Vorrichtung aus Fig. 2 in einem Schnitt entlang der Linie IV-IV;
- Fig. 5: eine alternative Ausführungsform mit innenliegendem Filter;
- Fig. 6: eine Ansicht wie Fig. 1 einer weiteren Ausführungsform;
- Fig. 7: die Vorrichtung aus Fig. 6 in einem Schnitt entlang der Linie VII-VII.

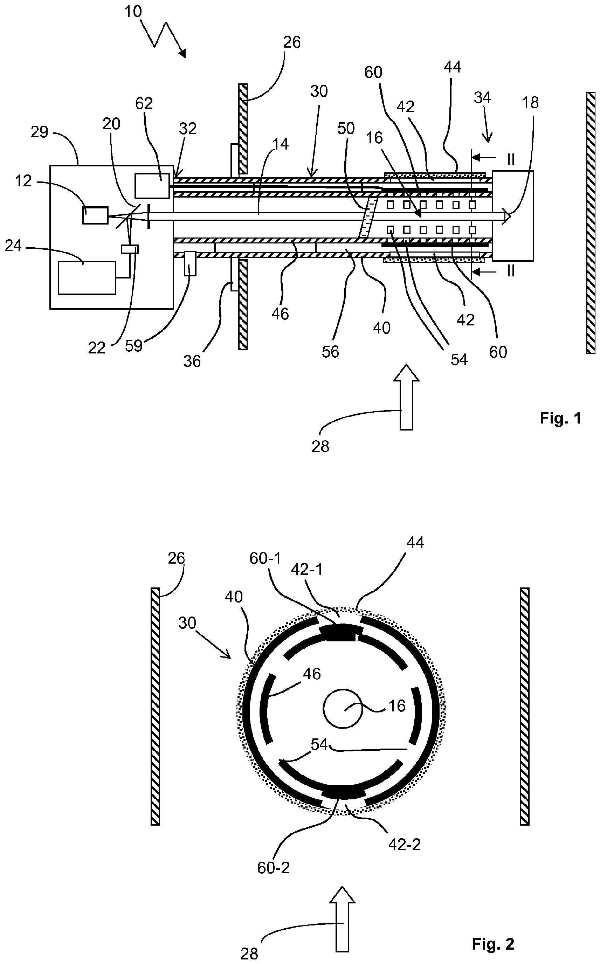

Eine erfindungsgemäße optoelektronische Vorrichtung 10 zur optischen in-situ Gasanalyse eines Gasstroms 28, der in einem Abgaskanal 26 geführt ist, weist in einem in Fig. 1 dargestellten ersten Ausführungsbeispiel einen Lichtsender 12 auf, der einen Sendelichtstrahl 14 aussendet. Der Sendelichtstrahl 14 definiert eine Messstrecke 16 und wird nach Reflexion an einem Retroreflektor 18 und einem Teilerspiegel 20 von einem Lichtempfänger 22 empfangen. Der Lichtempfänger 22 erzeugt in Abhängigkeit des auftreffenden Lichts Empfangssignale, die in einer Auswerteeinrichtung 24 ausgewertet werden, beispielsweise um die Konzentration einer Komponente des Messgases zu bestimmen.

Eine solche optoelektronische Vorrichtung 10 ist in diesem Ausführungsbeispiel als Transmissiometer ausgebildet, so dass mit dem Lichtempfänger 22 die Intensität des die Messstrecke 16 durchstrahlenden Lichts gemessen wird. In der Regel ist der Lichtsender 12 auf eine bestimmte Wellenlänge abgestimmt, die von einem zu untersuchenden Gasanteil, beispielsweise Schwefelwasserstoff, absorbiert wird. Über das am Lichtempfänger 22 empfangene Licht kann dann eine Aussage gemacht werden, wie hoch die Konzentration des interessierenden Gasanteils in dem Gasstrom 28 ist, der in dem Abgaskanal 26 geführt ist.

Die optoelektronische Vorrichtung 10 umfasst ein Gehäuse 29, mit einer Messlanze 30, dessen eine erste Ende 32 an das Gehäuse 29 angeschlossen ist und die mit ihrem anderen zweiten Ende 34 in den Abgaskanal 26 und damit in das zu messende Gas 28 hineinragt. Gehäuse 29 und Messlanze 30 sind über einen Befestigungsflansch 36 an einer Wand des Abgaskanals festgelegt.

In dem Gehäuse 29 sind die optoelektronischen Einheiten, wie Lichtsender 12, Lichtempfänger 22 und Auswerteeinrichtung 24 angeordnet, und in der Messlanze 30 ist das Licht durch die Messstrecke 16 geführt. An dem zweiten Ende 34 der Messlanze 30 ist der Retroreflektor 18 in einem Reflektorgehäuse gehalten.

Die Messlanze 30 weist ein Außenrohr 40 auf, dass sich über die gesamte Länge der Messlanze 30 erstreckt und an seinem einen Ende an dem Gehäuse 29 festgelegt ist und an seinem anderen Ende den Retroreflektor 18 hält. In dem Bereich des Außenrohres 40, der in den Abgaskanal 26 hineinragt, weist das Außenrohr 40 Öffnungen 42 auf, so dass Anteile des Gasstroms 28 in die Messstrecke 16 gelangen können.

Der in dem Abgaskanal 26 geführte Gasstrom 28, der lediglich durch einen Pfeil 28 angedeutet ist, kann mit Partikeln belastet sein, beispielsweise Staub, Rauch oder sonstige Aerosole, wobei die Partikel die eigentliche optische Messung auf der Messstrecke 16 stören. Um die Partikel von der Messstrecke 16 fern zu halten, ist zumindest im Bereich der Öffnungen 42 ein gasdurchlässiges Filter 44, bevorzugt aus porösem Material, vorgesehen. In dem Ausführungsbeispiel nach Fig. 1 und 2 befindet sich das Filter 44 auf der Außenseite des Außenrohres 40.

Weiter weist die Messlanze 30 in diesem ersten Ausführungsbeispiel ein vorzugsweise koaxial zum Außenrohr 40 angeordnetes Innenrohr 46 auf. Das Innenrohr 46 hat die gleiche Länge wie das Außenrohr 40. In dem Innenrohr 46 ist der Lichtstrahl 14 geführt. Das Innere des Innenrohres 46 ist durch ein dichtendes Fenster 50 in zwei Teile geteilt. In dem ersten Teil, der dem Gehäuse 29 zugewandt ist, kann kein Messgas und damit keine Verschmutzungen aus dem zweiten, dem Reflektor 18 zugewandten Teil gelangen. Im zweiten Teil, der sich am reflektorseitigen Ende des Innenrohres 46 befindet, weist das Innenrohr 46 Öffnungen 54 auf, durch die das Messgas 28 in die Messstrecke 16 gelangen kann.

Die Öffnungen 42 in dem Außenrohr 40 sind in diesem Ausführungsbeispiel als zwei größere Schlitzöffnungen 42-1 und 42-2 (Fig. 2) ausgebildet, durch die das Messgas 28 über den Filter 44 ein- und austreten kann. Durch die Öffnungen 54 in dem Innenrohr 46 kann das Messgas 28 dann bis in die Messtrecke 16 gelangen.

Die zwei Öffnungen 42-1 bzw. 42-2 des Außenrohres 40 lassen sich erfindungsgemäß mit jeweils einer Dichtung 60-1 bzw. 60-2 (nachfolgend auch einfach als "Dichtung 60" bezeichnet) verschließen. Die Dichtung 60 besteht aus einem in der Form der länglichen Öffnung 42 angepassten Streifen aus elastischem Material, wobei der Streifen in seinem Volumen veränderbar ist. Diese Volumenänderung kann pneumatisch oder hydraulisch oder in vergleichbarer Art erfolgen und von einem entsprechenden Aktor 62, zum Beispiel eine Pumpe, getrieben sein.

In einer Betriebsposition, bei der die reguläre Messung vorgenommen werden kann und bei der Messgas 28 in die Messstrecke 16 gelangen kann (Arbeitsbetrieb), befinden sich die Dichtungen 60 im nicht-expandierten Zustand und geben die Öffnungen 42 bzw. 42-1 und 42-2 frei. Dies ist in Fig. 1 und 2 dargestellt.

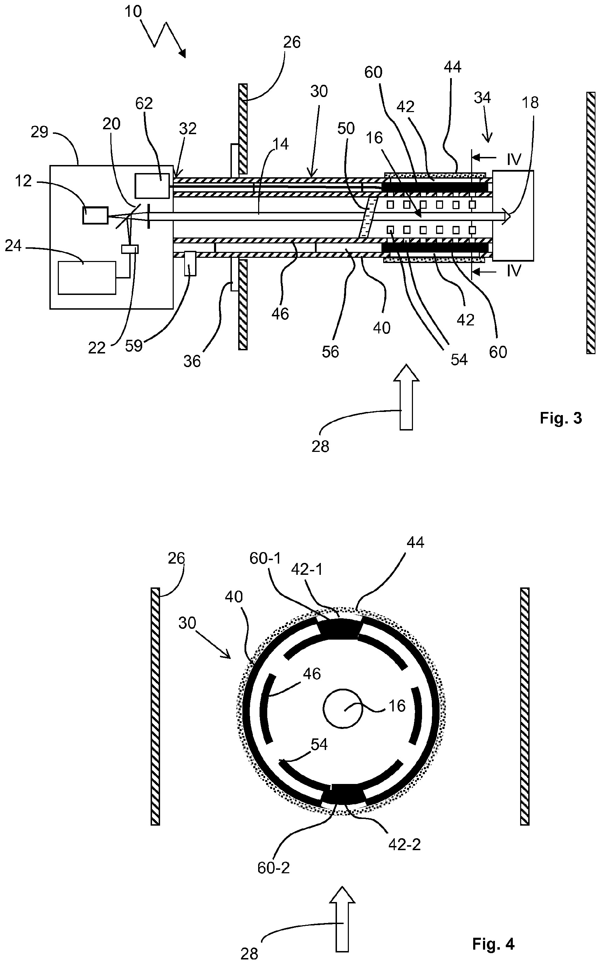

In einem Prüfbetrieb, in dem kein Messgas in die Messstrecke gelangen darf und die Messstrecke 16 frei von Messgas gehalten werden muss, damit ein Prüfbetrieb erfolgen kann, befinden sich die Dichtungen 60 im expandierten Zustand und verschließen die Öffnungen 42 bzw. 42-1, 42-2. Dies ist in den Fig. 3 und 4 dargestellt.

Um nach Verschließen der Öffnungen 42 die Messstrecke 16 frei von Messgas zu bekommen ist ein Prüfgasanschluss 59 vorgesehen, über den Prüfgas in Außenund Innenrohr geleitet werden kann, so dass das restliche Messgas aus der Messstrecke 16 verdrängt werden kann. Für eine Verdrängung ist es notwendig, dass so viel Prüfgas eingefüllt wird, dass der Druck in der Messstrecke 16 leicht höher ist als in dem Abgaskanal 26. Gleichzeitig ist eine definierte Undichtigkeit vorgesehen, so dass Messgas aus Innen- und Außenrohr und damit aus der Messstrecke 16 "gespült" wird.

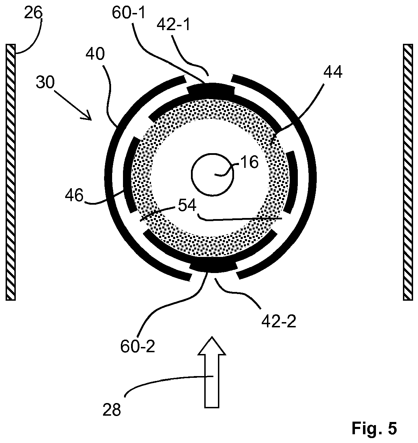

Es sind weitere konstruktive Alternativen möglich ohne den Grundgedanken der Erfindung zu verlassen, nämlich das Verschließen der Öffnungen 42 durch volumenveränderbare Dichtungen 60. Beispiele alternativer Konstruktionen sind in den Fig. 5 bis 7 gezeigt und nachfolgend kurz beschrieben. Dabei haben gleiche Teile wie in der ersten Ausführungsform gleiche Bezugszeichen und werden nicht nochmal beschrieben.

Fig. 5 zeigt eine ganz ähnliche Ausführungsform wie die aus den Fig. 1 bis 4, wobei hier die Abwandlung lediglich darin besteht, dass das Filter 44 noch innerhalb des Innenrohres 46 angeordnet ist.

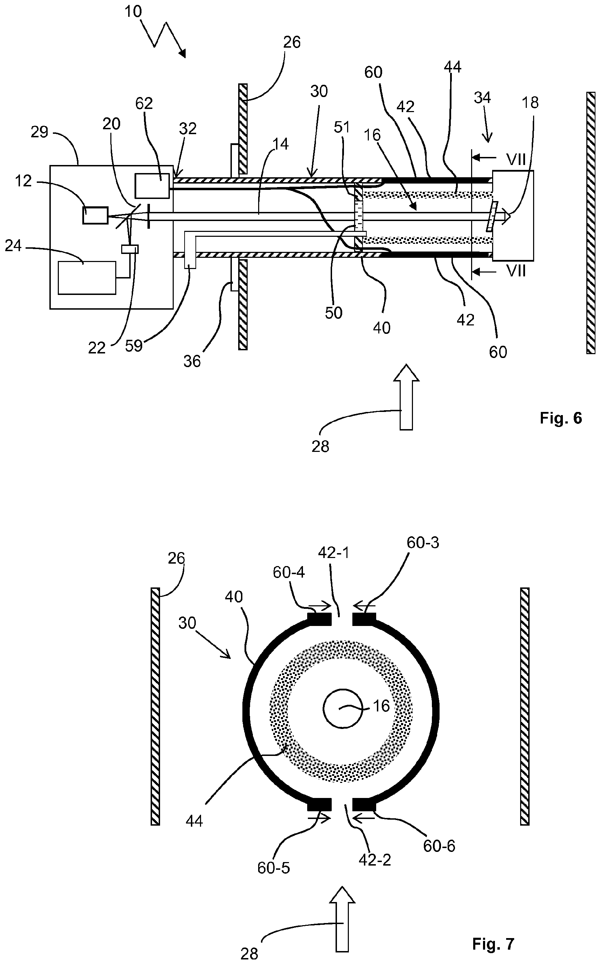

Eine weitere Ausführungsform ist in den Fig. 6 und 7 dargestellt. Dort gibt es kein Innenrohr, sondern lediglich das Außenrohr 40, dessen Rohrquerschnitt im Prinzip beliebig sein kann, z.B. rund, mehreckig, oval oder dergleichen. Die Messstrecke 16 liegt im Inneren des Filters 44, das in geeigneter Weise in dem Außenrohr 40 gehalten ist. Dies geschieht hier, indem das Filter 44 stirnseitig einerseits durch eine Wand 51, in der das Fenster 50 angeordnet ist, und andererseits durch das Reflektorgehäuse gehalten ist. Auf diese Weise ist auch sichergestellt, dass das Messgas nur durch das Filter 44 in die Messstrecke gelangen kann.

Die Dichtungen 60 sind jetzt, wie im Querschnitt der Fig. 7 zu erkennen, an dem Außenrohr 40 längsseits der Öffnungen 42-1 und 42-2 angeordnet. Es sind in diesem Beispiel für jede Öffnung zwei Dichtungen 60-3 und 60-4 bzw. 60-5 und 60-6 vorgesehen, die sich zum Schließen der Öffnung 42-1 bzw. 42-2 jeweils in Pfeilrichtung volumenmäßig vergrößern. Die Dichtungen 60-3 und 60-4 bzw. 60-5 und 60-6 sind wieder über den Aktor 62 ansteuerbar.

Wenn die Öffnungen 42-1 und 42-2 geschlossen sind, kann über den Prüfgasanschluss 59, der in diesem Ausführungsbeispiel bis in das Innere des Filters 44 verlängert ist, Prüfgas in das Messvolumen eingeleitet werden und das Messgas aus der Messtrecke 16 verdrängen.

Weitere konstruktive Alternativen sind denkbar. So müssen z.B. Filter und Messstrecke nicht unbedingt koaxial zum Außenrohr liegen. Sie könnten auch exzentrisch liegen. Wie erwähnt muss das Außenrohr im Querschnitt auch nicht rund sein. Es dient in erster Linie als Tragkonstruktion für Reflektorgehäuse, Filter und Messstrecke und als Trennmedium zum Gasstrom 28 im Abgaskanal 26.

## Patentansprüche

1. Vorrichtung zur optischen in-situ Gasanalyse, mit
- einem Gehäuse (29),
- einer Messlanze (30), deren eines erstes Ende (32) an das Gehäuse (29) angeschlossen ist und die mit ihrem anderen zweiten Ende (34) in das zu messende Gas (28) hineinragt,
- einem im Gehäuse (29) angeordneten Lichtsender (12), dessen Licht (14) in die Messlanze (30) geführt ist, und
- einem Lichtempfänger (22), der das von einem am zweiten Ende (34) angeordneten Reflektor (18) reflektierte Licht empfängt, wobei der Strahlengang eine optische Messtrecke (16) innerhalb der Messlanze (30) definiert,
- und einer Auswerteeinrichtung (24) zur Auswertung von Lichtempfangssignalen des Lichtempfängers (22),
- und einem Prüfgasanschluss (59), über den die Messstrecke (16) mit einem Prüfgas befüllbar ist,
- wobei die Messlanze (30) ein Außenrohr (40) aufweist und das Außenrohr (40) Öffnungen (42) für das zu messende Gas aufweist,
**dadurch gekennzeichnet,**
- **dass** die Öffnungen (42) mit wenigstens einer Dichtung (60) verschließbar sind,
- wobei die Dichtung (60) durch Vergrößerung ihres Volumens die Öffnungen (42) dichtend verschließt.

2. Vorrichtung nach Anspruch 1, **dadurch gekennzeichnet, dass** die Vorrichtung pneumatische oder hydraulische Mitteln umfasst, mit denen die Volumenänderung erfolgt.

3. Vorrichtung nach einem der vorhergehenden Ansprüche, **dadurch gekennzeichnet, dass** ein Innenrohr (46) in dem Außenrohr (40) angeordnet ist.

4. Vorrichtung nach einem der vorhergehenden Ansprüche, **dadurch gekennzeichnet, dass** in oder an der Messlanze (30) ein gasdurchlässiges Filter (44) angeordnet ist, durch das das Messgas in die Messstrecke (16) gelangt.

## Claims

1. Apparatus for optical in-situ gas analysis, comprising
- a housing (29),
- a measuring lance (30) having a first end (32) and a second end (34), with the first end (32) being connected to the housing (29), with the second end (34) projecting into the gas (28) to be measured,
- a light transmitter (12) that is arranged in the housing (29) and with the light (14) from the light transmitter (12) being conducted into the measuring lance (30),
- and a light receiver (22) which receives the light which is being reflected by a reflector (18) arranged at the second end (34), with the optical path defining an optical measurement path (16) within the measuring lance (30),
- and an evaluation device (24) for evaluating received light signals of the light receiver (22),
- and a test gas connection (59), with the measurement path (16) being able to be filled with a test gas via the test gas connection (59),
- whereby the measuring lance (30) has an outer tube (40) and the outer tube (40) has openings (42) for the gas to be measured;
**characterized in that**
- the openings (42) can be closed by at least one seal (60);
- wherein the seal (60) sealingly closes the openings (42) by enlarging its volume.

2. Apparatus in accordance with claim 1, **characterized in that** the apparatus is configured to bring about the enlargement of the volume of the at least one seal by using pneumatic or hydraulic means.

3. Apparatus in accordance with any preceding claim, **characterized in that** an inner tube (46) is arranged in the outer tube (40).

4. Apparatus in accordance with any preceding claim, **characterized in that** a gas-permeable filter (44) is arranged in or at the measuring lance (30), with the measuring gas entering into the measurement path (16) via the gas-permeable filter (44).

## Revendications

1. Appareil d'analyse optique de gaz in situ, comprenant
- un boîtier (29),
- une lance de mesure (30) dont une première extrémité (32) est reliée au boîtier (29), et avec la seconde extrémité (34) surplombe le gaz (28) à mesurer,
- un émetteur de lumière (12) qui est agencé dans le boîtier (29) dont la lumière (14) est conduite dans la lance de mesure (30),
- un récepteur de lumière (22) qui reçoit la lumière qui est réfléchie par un réflecteur (18) disposé à la seconde extrémité (34), et le chemin optique définit un trajet de mesure optique (16) à l'intérieur de la lance de mesure (30),
- et un dispositif d'évaluation (24) pour évaluer les signaux lumineux reçus du récepteur de lumière (22),
- et une connexion de gaz d'essai (59), à travers le trajet de mesure (16) peut être rempli d'un gaz d'essai,
- la lance de mesure (30) comportant un tube externe (40) et le tube externe (40) comporte des ouvertures (42) pour le gaz à mesurer;
**caractérisé en ce que**
- les ouvertures (42) peuvent être fermées par au moins un joint d'étanchéité (60);
- et le joint d'étanchéité (60) ferme les ouvertures (42) de manière étanche en augmentant son volume.

2. Appareil selon la revendication 1, **caractérisé en ce que** l'appareil comprend des moyens pneumatiques ou hydrauliques avec lesquels le changement de volume a lieu.

3. Appareil selon l'une des revendications précédentes, **caractérisé en ce qu'**un tube interne (46) est disposé dans le tube externe (40).

4. Appareil selon l'une des revendications précédentes, **caractérisé en ce qu'**un filtre perméable aux gaz (44) est disposé dans ou à la lance de mesure (30), à travers le gaz de mesure pénétrant dans le trajet de mesure (16).
